# EUROPEAN PATENT APPLICATION

(11) **EP 1 298 210 A1**
(43) Date of publication of application: **02.04.2003**
(21) Application number: 01123584.3
(22) Date of filing: 01.10.2001
(51) Int. Cl.: C12N 15/29, C07K 14/415, C07K 4/10, A23G 1/02

(54) **Cocoa flavour precursor peptides**

(71) Applicant: SOCIETE DES PRODUITS NESTLE S.A., 1800 Vevey (CH)
(72) Inventor: Kochhar, Sunil, 1073 Savigny (CH); Hansen, Carl Erik, 1066 Epalinges (CH); Juillerat, Marcel Alexandre, 1000 Lausanne 26 (CH); Wille, Hans-Juergen, York Y03 6GY (GB); Buyukpamukcu, Elif, 1005 Lausanne (CH); Keely, Brendan, York Y023 2QH (GB); Goodal, David Murray, York Y032 9RB Huntington (GB)
(74) Representative: Becker Kurig Straus

(57) **Abstract**

The present invention pertains to peptides derived from fermented cocoa beans and representing cocoa and/or chocolate flavour precursors. In particular, the present invention relates to a process for preparing cocoa and/or chocolate flavour precursor peptides from fermented cocoa beans and to the preparation of cocoa/chocolate flavour on a synthetic basis, and to the use thereof in the preparation of chocolate.

## Description

The present invention pertains to peptides derived from fermented cocoa beans and representing cocoa and/or chocolate flavour precursors. In particular, the present invention relates to a process for preparing cocoa and/or chocolate flavour precursor peptides from fermented cocoa beans and to the preparation of cocoa/chocolate flavour on a synthetic basis, and to the use thereof in the preparation of chocolate.

The processing of cocoa beans comprises the basic steps of fermentation, drying and roasting.

During fermentation the cocoa seed proteins are degraded by microbiological and enzymatic processes to mainly hydrophilic peptides and hydrophobic amino acids, which serve as flavour precursors for the unique chocolate flavour. The cocoa proteolytic system involved is highly efficient. The endogenous proteolytic activity in cocoa is represented by endo-proteases, aminopeptidases and carboxypeptidases, which in concert have the capability to hydrolyze intact protein to single amino acids and oligopeptides of different length.

During roasting various chemical reactions occur, in particular Maillard-type reactions and thermal degradation reactions (decarboxylations, deaminations, oxidations etc.). The Maillard reaction involving peptides, amino acids and reducing sugars generates compounds that are deemed to eventually contribute to the final cocoa/chocolate flavour.

In the recent past more and more evidence has been brought up that the size of the peptides and their amino acid contents/sequence play an important role in flavour formation. The amino acid pool in un-roasted and roasted fermented cocoa has been investigated and certain hydrophobic amino acids have been found to be implicated in the generation of cocoa flavour (Seiki, Rev. Int. Choc. **28** (1973) 38-42). In addition several attempts to produce artificially cocoa flavour are known in the art, such as e.g. subjecting acetone dried powder prepared from unfermented ripe cocoa beans to an autolysis at a pH of 5.2 followed by roasting in the presence of reducing sugars (Voigt et al., Food Chem. **49** (1994), 173-180).

However, little is known in the art about short-chain peptides present in the cocoa peptide pool. So far, merely four major proteins with an apparent molecular weight of 14.5, 21, 31 and 47 kDa, respectively, could be identified in cocoa beans before fermentation, which proteins are deemed to be the main source for the peptide/amino acid pool, generated during fermentation.

One of the major limitations in studying the peptide pool in fermented cocoa beans is the presence of UV absorbing compounds, e.g. theobromine, caffeine, epicatechin, or polyphenols in general. Extraction of the proteinaceous content in the presence of such compounds generally leads to extensive complexation. As a consequence, trying to remove said polyphenols with current solvent systems, such as aqueous acetone or methanol, was not successful, as these solvent systems also remove low-molecular-peptides.

Since so far no substantial information about short-chain peptides present in fermented cocoa beans is presently available an object of the present invention resides in providing more detailed data about said peptides and eventually provide means for improving the preparation of cocoa flavour.

The above object has been solved by providing peptides obtainable by a process comprising the steps of
(i) preparing a cocoa nib powder starting from fermented cocoa beans;
(ii) extracting said cocoa nib powder with aequous acetic acid (50 %);
(iii) separating non-proteinaceous compounds with a solid phase adsorption and collecting the elute containing peptides;
(iv) diluting the elute with 5 volumes of 0.1 % trifluoroacetic acid;
(v) loading the diluted elute on a RP-HPLC column equilibrated with 0.14 % sodium acetate/0.05 % TEA and eluting with an increasing concentration of 80 % acetonitrilie/0.1 % trifluoracetic acid;
(vi) collecting the elutes containing the peptides

The peptides according to the present invention have been shown to exhibit a size of approximately 2 to 25 amino acids and may serve as precursors for flavour active substances.

In the figures,
Fig. 1 shows an evaluation of different solvents to extract proteinaceous material from fermented cocoa beans;
Fig. 2 shows the results of RP-HPLC purification as determined by UV absorbance (A) and a fluorimetric assay (B,C).

During the extensive studies leading to the invention, the present inventors have designed novel methods for the isolation of short-chain cocoa peptides derived from fermented cocoa beans.

The peptides or peptide pool derived from fermented cocoa beans of the present invention are obtainable by a process comprising the above steps steps (i) to (iv).

During step (i) fermented cocoa beans are crushed by conventional means, such as a universal mill with the grinded material being preferably sieved to remove remaining large particles.

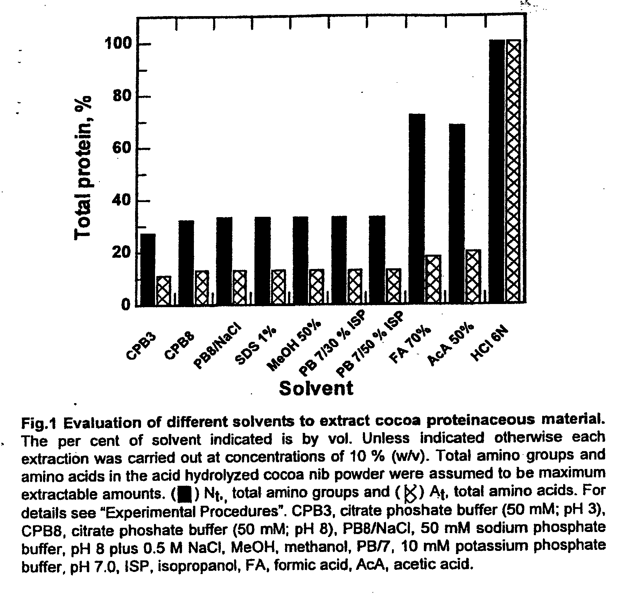

In order to be capable to primarily extract the peptides of interest extensive studies have been carried out to find an appropriate solvent system. To this end, several solvent systems have been investigated involving citrate phosphate buffer (50mM; pH 3), citrate phosphate buffer (50mM; pH 8), 50 mM sodium phosphate buffer, pH 8 plus 0.5 M NaCl, methanol, 10 mM potassium phosphate buffer, pH 7.0, isopropanol, formic acid and acetic acid (Fig. 1).

Acetic acid (50%) provided an overall recovery of total amino groups of about 70 % with the additional advantage of its easy removal under low pressure and its ability to inhibit oxidation reactions. Moreover, it has been found that by using this particular solvent there is no need to carry out laborious and time consuming processes of fat removal with lipohilic solvents prior to extraction, such that both intact or defatted cocoa nib powder may be used.

In order to provide best results the cocoa nib powder is suspended in the solvent, sonicated and optionally further homogenized by appropriate means. The suspension may be cooled on ice and centrifuged at 15.000 rpm at 4 °C for 15 min to obtain a clear supernatant between the pellet and the fat layer at the surface. For further purification this supernatant may be separated, passed through a 0.22 µm filter disc and optionally kept at-20 °C until further processing.

During step (iii) the acetic acid extract obtained is subjected to a treatment to remove non-proteinaceous, organic compounds, in particular UV-absorbing compounds and polyphenols, respectively. This may be achieved by means of a solid phase extraction with subsequent centrifugation of the elute and isolation of the supernatant solution. To this end a Chromabond® cartridge (obtainable from Macherey-Nagel) equilibrated with 0.1 % TFA (trifluoracetic acid) may be used on which the extract is loaded. For differentially eluting the adsorbed material from the column a solvent system consisting of 70 % methanol in 0.1 % trifluoracetic acid may be used. However, also other solvent systems may be put to use with the results of the elution being monitored via conventional means comprising mass spectrometry, UV/VIS or fluorometric assays in combination with UV/VIS.

The respective elute(s) comprising the peptides of interest is(are) then dried under reduced pressure to remove the solvent and is subsequently dissolved in an appropriate solvent for further processing, such as 0.1 % TFA or 25 % acetonitrile/0.1% TFA (v/v).

Following, the solution obtained as above is diluted with about 5 volumes of 0.1 % trifluoracetic acid and is loaded on a RP-HPLC column equilibrated with 0.14 % sodium acetate/0.05 % TEA. The adsorbed material is eluted with increasing concentrations of a solvent comprising 80 % acetonitrilie/0.1 % trifluoracetic acid.

Finally, the elutes containing the objective peptides are collected and optionally dried and dissolved in an appropriate solvent.

The peptides obtainable according to the above described methods have a size of between about 2 to 25 amino acids. In order to obtain compounds exhibiting a cocoa and/or chocolate flavour, these peptides obtained as above may be subjected to a Maillard reaction involving reducing sugars or may be subjected to a further enzymatic degradation using e.g. aspartic endoproteinase, cysteine endoproteinase, carboxypeptidase. Consequently, the peptides of the present invention or the products or fragments thereof may serve as precursors of flavour-active compounds. The flavour-active substances obtainable according to the present invention may be added to food products, cosmetic products and/or pharmaceutical products.

The present invention also pertains to a process for obtaining cocoa/chocolate flavour precursors, which process comprises the steps (i) to (vi) as detailed above.

The following examples illustrate the invention in a more detailed manner. It is, however, understood that the present invention is not limited to the examples but is rather embraced by the scope of the appended claims.

### Example 1

### Preparation of fermented cocoa nib powder

West African Amelonado cocoa beans were fermented in 80 kg heaps covered with banana leaves in Ivory Coast. Samples were removed at 1 day intervals during 7 day fermentation period, sun dried, hand peeled and shipped. The cocoa beans were kept at -20 °C.

Dried cocoa beans were passed through a bean crusher followed by a winnover to remove shells. Cocoa nibs were milled for a few seconds in an universal mill. The nib powder was passed through 0.8-mm sieve and kept at 4 °C. Unless stated otherwise, the fermented beans used throughout the present study were 5 day Amelonado beans.

### Preparation of unfermented cocoa nib powder

Cacoa nib powder was prepared form unfermented cocoa beans in an analogous manner.

### Extraction of Cocoa nib powder

Cocoa nib powder (2 g) was suspended in 20 ml of a solvent system [citrate phosphate buffer (50mM; pH 3), citrate phosphate buffer (50mM; pH 8), 50 mM sodium phosphate buffer, pH 8 plus 0.5 M NaCl, methanol, 10 mM potassium phosphate buffer, pH 7.0, isopropanol, formic acid (70 %) and acetic acid (50 %)]. The resulting suspension was sonicated for 1 min at full speed (10 mm probe at medium setting and full power, Labsonic U, B. Braun). The suspension was then further subjected to homogenization treatment for 1 min at a maximum speed (Polytron, Kinematica AG). The suspension was cooled on ice for 15-30 min and centrifuged at 15.000 rpm at 4 °C for 15 min. The clear supernatant located between the pellet and the fat layer at the surface was carefully removed, passed through 0.22 µm filter disc and kept at -20 °C until further processing.

In order to determine the effect of the extraction procedure the amount of amino groups present in the sample of the fermented CNP as well as in the extracts were determined by acid hydrolysis (6N HCl at reduced pressure at 110 °C for 24 hrs).

To this end, aliquots of the respective samples (10 µl) were dried under reduced pressure in Pyrex glass tubes and subsequently acid hydrolyzed under vapor phase of HCl using Waters Pico Tag Workstation equipped with Savant SpeedVac concentrator (refrigerated trap RVT 400; SVC 100 H centrifuge and Alc-Tel vacuum pump). Samples were flushed with nitrogen followed by applying reduced pressure until few air bubbles were visually detected. The process was repeated 5-times and the tubes were sealed under reduced pressure and placed at 110 °C for 24 hrs. After hydrolysis, samples were brought to room temperature and the vacuum applied was carefully bled, to avoid splashing of HCl. The excess HCl was removed and the hydrolyzed samples were dried under reduced pressure after the addition of 20 µl of re-dry buffer (water/methanol/TEA (triethylamine); 2:2:1) and suspended in 200 µl of water.

The hydrolyzed samples (10 µl) were dried in a SpeedVac and dissolved in 10 µl re-dry buffer and again dried. The dried samples were dissolved in 20 µl of derivatization solution (ethanol/water/TEA/PITC (phenylisothiocyanate); 7:1:1:1), vortexed and incubated at room temperature for 10 minutes. The samples were dried in a rotary SpeedVac for 60 minutes (reduced pressure 50-60 m Torr) and dissolved in 100 µl solvent A (0.14 M Na-acetate/0.05% TEA) and subjected to RP-HPLC for PITC derivatized amino acid analysis (Heirikson and Meredith Anal. Biochem. **136** (1984), 65-74).

The RP-HPLC was carried out on Hewlett Packard 1050 HPLC system, with the detection being carried out with an UV/VIS diode array detector. The solvents were degassed on-line using Gastorr G-104 automatic degassers. The derivatized amino acids (10 µl) were injected on to reverse-phase C18 column (AccQ.Tag column (3.9x150mm) from Waters) and the amino acid derivatives were eluted with an increasing linear gradient of solvent B (60 % v/v acetonitrile) in solvent A (supra) as follows: 0-48 % B in 10 min; 48 - 100 % in 0.2 min followed by isocratic elution at 100 % B for 1.5 min. Peaks were recorded and integrated by HP chemstation software.

It could be shown that intact proteins and peptides were best dissolved in acetic acid and formic acid, which gave a 2-fold better recovery as compared to the other solvent systems. Due to its ease of handling acetic acid has been chosen.

### Example 2

### Separation of the amino acid/peptide pool from polyphenols

In order to separate the short-chain peptides from other organic compounds solid phase extraction employing Chromabond® C8 cartridge (500 mg) has been utilized. Acetic acid extracts as obtained in example 1 were diluted 5-times with water, chilled on ice for 15 min and centrifuged (5.000 rpm) for 15 min at 4 °C yielding a clear supernatant. Chromabond® cartridge was pre-conditioned by passing 1 ml of methanol followed by 2 ml 50 % methanol in 0.1 % TFA. The cartridge was equilibrated with 3 ml 0.1 % TFA. The sample (1 ml) was passed through the column slowly followed by 2-times 1 ml wash with 10 % methanol in 0.1 % TFA. The adsorbed material from the column was eluted with 2-times 1 ml of 70 % methanol on 0.1 % TFA. The column wash and 10 % methanol elute fractions were pooled (SPE elute A), dried under low pressure and redissolved in 0.1 % TFA. The 70 % elute fraction (SPE elute B) was also dried under low pressure and redissolved in 25 % ACN (acetonitrile)/0.1 %TFA.

The determination of the total primary amino groups and amino acid analysis before and after acid hydrolysis (supra, example 1) showed that SPE elute A represents over 90 % of the total soluble proteinaceous material (Table 1). SPE elute A from acetic acid extract of fermented beans showed a 3-fold increase following acid hydrolysis indicating presence of peptides (Table 1). The average chain length of peptides was 3 amino acid residues.

**Table 1**

| | Amino groups | | Recovery, % | |
|---|---|---|---|---|
| | N_{α}^{a} | Nₜ^{b} | N_{α}^{a} | Nₜ^{b} |
| | *µmol* | | | |
| Unfermented | | | | |
| Supernatant | 35 | 38 | 100 | 100 |
| SPE elute A | 29 | 26 | 83 | 68 |
| SPE elute B | 1 | 6 | 3 | 16 |
| | | | | |

| Fermented | | | | |
|---|---|---|---|---|
| Supernatant | 90 | 218 | 100 | 100 |
| SPE elute A | 68 | 127 | 76 | 58 |
| SPE elute B | 11 | 81 | 12 | 37 |

| | | | | |
|---|---|---|---|---|
| ^{a} Before and | | | | |
| ^{b} after acid hydrolysis (6N HCl at 110 °C for 24 h). N_{α} and Nₜ, Free and total amino groups. | | | | |

### Example 3

### Separation of peptides by HPLC

Short-chain hydrophilic peptides and the majority of amino acids are in general not retained 5 on a reverse-phase HPLC column due to their poor interactions with the hydrophobic chains (C8 or C18) of the stationary phase. The interactions of hydrophilic peptides and amino acids can even be improved by the inclusion of aliphatic sulfonic acids, e.g. hexane- or heptane-sulfonic acid. In the case of ion-pair RP-HPLC, 5 mM sodium hexane sulfonate was included in solvent A and B.

Samples (20 µl) were injected onto the RP-column (Spherisorb 80-5C8 (250 x 4.6 mm)) and eluted with a solvent system comprising solvent A (0.1 % v/v TFA in water), to which hexanesulfonic acid has been added (*5mM*) and solvent B (80 % v/v CAN and 0.1 % v/v TFA), also including hexanesulfonic acid in the same amount, with isocratic at 0 % solvent B for 10 minutes, 0-50 % B in 60 minutes, isocratic at 50 % B for 5 min, 50-100 % B in 25 minutes and isocratic at 100 % B for 5 minutes.

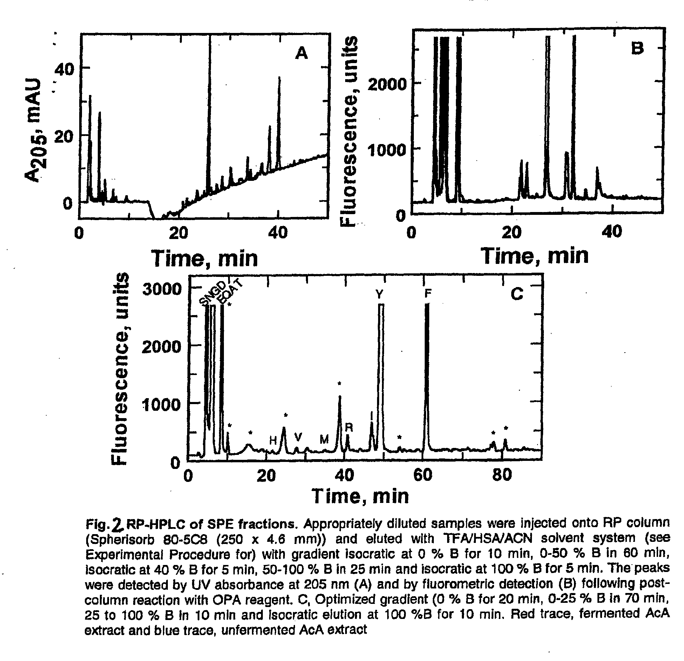

The peaks were detected by UV absorbance at 205 nm (fig. 2A) and by fluorometric detection following post column reaction with a o-phthaldialdehyde (OPA) reagent (Fig. 2B) (Jarrett et al. Anal. Biochem. 153 (1986), 189-198).

An optimized gradient (0 % B for 20 min, 0-25 % B in 70 min; 25 - 100 % B in 10 min; and isocratic elution at 100 % B for 10 min) gave best results as may be seen in Fig. 2C.

The same assay as above was repeated without the addition of hexanesulfonic acid and the elutes during the first 10 minutes were analyzed by electrospray ionization (ESI) mass spectrometry using a FinniganMat LCQ mass spectrometer interfaced with an HPLC (Spectra system, FinniganMat). The HPLC system (Spectra system, FinniganMat) interfaced with LCQ mass spectrometer (FinniganMat) consisted of a quarternary pump (TSP P4000), an autoinjector (TSP AS3000) and a UV/VIS detector (UVIS 205 from Linear Instruments) equipped with high pressure stainless steel flow cell (1.6 µl volume, 2 mm path-length). LC flow was directed to LCQ mass spectrometer without using a flow-splitter. Typically the following conditions were used: capillary temperature, 200°C, sheath gas flow, 70; auxiliary gas flow, 10; source voltage, 5kV. Other parameters were adjusted automatically during calibration /tuning procedure as recommended by the manufacture.

A comparison of molecular ions data [(M+H)]⁺ from fermented and non fermented cocoa beans showed the presence of 43additional molecular ions in the fermented beans elute.

Each identified molecular mass ion was upon MS/MS analysis, and in conjunction with on-line data analysis using Sequest ™ database, identified various peptides- both of cocoa vicillin and albumin origin. 18 peptide sequences from vicillin and 25 peptide sequences from albumin, a total of 43 sequences, were observed.

**Table 3**

| Masses of peptides deduced using Sequest ™ | | |
|---|---|---|
| **Peptide, *m*/*z*** Molecular ion | **Sequence** | **Source protein** |
| 698.4 | PVNSPGK | Vicilin |
| 861.4 | PVNSPGKY | Vicilin |
| 1118.5 | NADSKDDVVR | Albumin |
| 1205.6 | SNADSKDDVVR | Albumin |
| 1391.6 | SNADSKDDVVRVS | Albumin |
| 629.3 | VPHYN | Vicilin |
| 515.3 | VPHY | Vicilin |
| 784.4 | ENSPPLK | Vicilin |
| 855.4 | AENSPPLK | Vicilin |
| 727.4 | AENSPPL | Vicilin |
| 600.3 | KDQPL | Vicilin |
| 872.4 | ASKDQPLN | Vicilin |
| 758.4 | ASKDQPL | Vicilin |
| 487.2 | KAGVL | Albumin |
| 544.3 | KAGVLG | Albumin |
| 857.5 | KIEKAGVL | Albumin |
| 431.2 | AGGGGL | Albumin |
| 688.3 | ISGAGGGGL | Albumin |
| 933.4 | SSISGAGGGGLA | Albumin |
| 862.5 | SSISGAGGGGL | Albumin |
| 838.4 | DEEGNFK | Vicilin |
| 373.3 | KIL | Vicilin |
| 710.3 | DEEGNF | Vicilin |
| 300.2 | APL | Vicilin |
| 621.3 | SPGDVF | Vicilin |
| 902.5 | APLSPGDVF | Vicilin |
| 826.4 | RNNPYY | Vicilin |
| 1042.5 | NNPYYFPK | Vicilin |
| 940.4 | YDNSAGKW | Albumin |
| 1054.5 | NYDNSAGKW | Albumin |
| 892.4 | DTDGDELQ | Albumin |
| 1050.4 | DTDGDELQTG | Albumin |
| 715.4 | ANSPVLD | Albumin |
| 600.3 | ANSPVL | Albumin |
| 563.3 | DNEW | Albumin |
| 820.3 | DNEWAW | Albumin |
| 475.3 | PVIF | Albumin |
| 576.3 | TPVIF | Albumin |
| 747.4 | NGTPVIF | Albumin |
| 555.3 | AGHAVT | Vicilin |
| 453.2 | DYR | Vicilin |
| 690.4 | NGKGTIT | Vicilin |
| 403.2 | DRL | Albumin |
| 484.3 | VPIR | Albumin |
| 822.4 | SNQNGRF | Vicilin |
| 460.3 | DIGR | Albumin |
| 634.3 | VSTDVN | Albumin |
| 453.2 | DYR | Vicilin |
| 1367.9 | PAGNNKPESYYGA | Vicilin |
| 328.2 | VPI | Albumin |
| 435.3 | RIF | Vicilin |
| 784.4 | GEPGPNTL | Albumin |

### Example 4

### Roasting experiments

In a subsequent step, it was determined which peptides and amino acids are potentially yielding flavour active substances, when subjected to a reaction during the roasting step.

To this end, fermented and dried cocoa beans were roasted at 145 °C for 30 min and in addition one sample for each day of fermentation was prepared. The roasted beans were ground and extraction was carried out. For comparison, non-roasted beans were processed at the same time as roasted beans. LC-MS/MS carried out on both roasted and non-roasted beans.

As a result, it was observed that nearly all peptides from vicilin and albumin fragmentation appear after roasting, but a substantial reduction in their quantity could be observed.

## Claims

**1.** Cocoa and/or chocolate flavour precursor peptides obtainable by a process comprising the steps of:
(i) preparing a cocoa nib powder starting from fermented cocoa beans;
(ii) extracting said cocoa nib powder with aequous acetic acid (50 %);
(iii) separating non-proteinaceous compounds with a solid phase adsorption and collecting the elute containing peptides;
(iv) diluting the elute with 5 volumes of 0.1 % trifluoroacetic acid;
(v) loading the elute fraction on a RP-HPLC column equilibrated with 0.14 % sodium acetate/0.05 % TEA and eluting with an increasing concentration of 80 % acetonitrile/0.1 % trifluoracetic acid;
(vi) collecting the elutes containing the peptides

**2.** The peptides according to claim 1, wherein the peptides have a size of about 2 to 30 amino acids, preferably 2 to 5 amino acids.

**3.** A process for preparing cocoa and/or chocolate flavour, which comprises, subjecting the peptides obtained according to claim 1 or 2 to a Maillard reaction with reducing sugars.

**4.** The process according to claim 3, which comprises the step of further digesting the peptides obtained according to a process according to claim 1 with proteases.

**5.** Use of a peptide according to any of the claims 1 or 2 for the preparation of cocoa and/or chocolate flavour.

**7.** Use of a peptide according to any of the claims 1 or 2 for the preparation of confectionery, ice cream, beverages, dairy products, cosmetic products, pet food or pharmaceutical products.

**8.** The use according to claim 7, wherein the confectionery product is chocolate.
